# EUROPÄISCHE PATENTSCHRIFT

(11) **EP 3 186 222 B1**
(45) Veröffentlichungstag und Bekanntmachung des Hinweises auf die Patenterteilung: **06.06.2018**
(21) Anmeldenummer: 15753047.8
(22) Anmeldetag: 20.08.2015
(51) Int. Cl.: C07C 217/08, B01D 53/14, C10L 3/10, B01J 31/24

(54) **DIAMIN MIT TERT-ALKYLAMINO- UND PRIMÄRER AMINOGRUPPE ZUR VERWENDUNG IN DER GASWÄSCHE**
DIAMINE WITH TERTIARY ALKYL-AMINO AND PRIMARY AMINO GROUP FOR USE IN GAS SCRUBBING
DIAMINE AVEC UN GROUPE TERT-ALKYLAMINE ET AMINO PRIMAIRE POUR L'UTILISATION DANS LE LAVAGE DE GAZ

(30) Priorität: 25.08.2014 EP 14182096
(43) Veröffentlichungstag der Anmeldung: 05.07.2017
(73) Patentinhaber: BASF SE, 67056 Ludwigshafen am Rhein (DE)
(72) Erfinder: INGRAM, Thomas, 68163 Mannheim (DE); DA SILVA, Marion, 68165 Mannheim (DE); SIEDER, Georg, 67098 Bad Dürkheim (DE)
(86) Internationale Anmeldenummer: PCT/EP2015/069164
(87) Internationale Veröffentlichungsnummer: WO 2016/030277

(56) Entgegenhaltungen:
- WO-A1-2009/137765
- WO-A1-2013/138443
- DE-A1- 2 628 376
- US-A- 4 336 233
- US-A- 4 471 138
- US-A- 4 894 178

## Beschreibung

Die vorliegende Erfindung betrifft bestimmte Diamin-Verbindungen, diese Verbindungen enthaltende Absorptionsmittel, die Verwendung dieser Absorptionsmittel zum Entfernen von Kohlendioxid und/oder Schwefelwasserstoff aus Fluidströmen und Verfahren zum Entfernen von Kohlendioxid und/oder Schwefelwasserstoff aus Fluidströmen.

Die Entfernung von Sauergasen, wie z. B. CO₂, H₂S, SO₂, CS₂, HCN, COS oder Merkaptanen, aus Fluidströmen wie Erdgas, Raffineriegas oder Synthesegas, ist aus unterschiedlichen Gründen von Bedeutung. CO₂ kann in Verbindung mit Wasser, welches in den Fluidströmen häufig mitgeführt wird, Säuren bilden, welche zur Korrosion an Leitungen und Armaturen führen. Unter anderem aus Erdgas muss Kohlendioxid so weit entfernt werden, dass der Brennwert des Gases nicht unter den gewünschten Wert fällt. Für die Weiterverarbeitung in einer Erdgasverflüssigungsanlage (LNG = liquefied natural gas) muss CO₂ hingegen vollständig entfernt werden.

Der Gehalt an Schwefelverbindungen von Erdgas muss durch geeignete Aufbereitungsmaßnahmen unmittelbar an der Erdgasquelle reduziert werden, denn die Schwefelverbindungen bilden in dem vom Erdgas häufig mitgeführten Wasser Säuren, die korrosiv wirken. Für den Transport des Erdgases in einer Pipeline oder die Weiterverarbeitung in einer Erdgasverflüssigungsanlage (LNG = liquefied natural gas) müssen daher vorgegebene Grenzwerte der schwefelhaltigen Verunreinigungen eingehalten werden. Darüber hinaus sind zahlreiche Schwefelverbindungen bereits in niedrigen Konzentrationen übel riechend und toxisch.

Zur Entfernung von Sauergasen werden Wäschen mit wässrigen Lösungen anorganischer oder organischer Basen eingesetzt. Beim Lösen von Sauergasen in dem Absorptionsmittel bilden sich mit den Basen Ionen. Das Absorptionsmittel kann durch Entspannen auf einen niedrigeren Druck und/oder Strippen regeneriert werden, wobei die ionischen Spezies zu Sauergasen zurück reagieren und/oder mittels Dampf abgestrippt werden. Nach dem Regenerationsprozess kann das Absorptionsmittel wiederverwendet werden.

Hohe CO₂-Absorptionsgeschwindigkeiten werden durch die Verwendung von Absorptionsmitteln mit einer hohen CO₂-Affinität, wie primären und sekundären Alkanolaminen erreicht. Die hohe CO₂-Affinität bedingt, dass die CO₂-Absorption stark exotherm verläuft. Derartige Absorptionsmittel erfordern allerdings aufgrund des hohen Betrags der Absorptionsreaktionsenthalpie in der Regel auch einen höheren Energieverbrauch bei der Regeneration.

Stark sterisch gehinderte sekundäre Amine, wie 2-(2-tert-Butylaminoethoxy)ethanol, und tertiäre Amine, wie Methyldiethanolamin (MDEA), zeigen kinetische Selektivität für H₂S gegenüber CO₂. Diese Amine reagieren nicht direkt mit CO₂; vielmehr wird CO₂ in einer langsamen Reaktion mit dem Amin und mit Wasser zu Bicarbonat umgesetzt - im Gegensatz dazu reagiert H₂S in wässrigen Aminlösungen sofort. Diese Amine eignen sich daher insbesondere für eine selektive Entfernung von H₂S aus Gasgemischen, die CO₂ und H₂S enthalten.

Sterisch ungehinderte primäre oder sekundäre Amine, beispielsweise Piperazin, können als Promotoren die CO₂-Absorption von tertiären Aminen durch intermediäre Bildung einer Carbamatstruktur beschleunigen. Bei dieser direkten Reaktion des Amins mit Kohlendioxid ist die Absorptionsgeschwindigkeit hoch, dafür kann jedoch nur ein CO₂-Molekül von zwei Amin-Molekülen aufgenommen werden. So offenbart die US 4,336,233 ein Verfahren zur Entfernung von CO₂ und/oder H₂S aus Gasen mittels eines wässrigen Absorptionsmittels, welches MDEA und Piperazin enthält. Die Verwendung von Piperazin als CO₂-Promotor ermöglicht eine um ein vielfaches höhere CO₂-Absorptionsgeschwindigkeit gegenüber Systemen ohne Promotor.

Die US 2013/0243676 beschreibt ein Verfahren zur Absorption von H₂S und CO₂ aus einem Gasgemisch mit einem Absorptionsmittel, welches einen stark sterisch gehinderten tertiären Etheramintriethylenglykolalkohol oder Derivate davon sowie ein flüssiges Amin umfasst.

In der US 4,471,138 wurde gezeigt, dass stark sterisch gehinderte sekundäre Amine, wie 2-(2-tert-Butylaminoethoxy)ethanol (TBAEE), auch in Kombination mit weiteren Aminen wie Methyldiethanolamin (MDEA), eine deutlich höhere H₂S-Selektivität als MDEA aufweisen. Als stark sterisch gehindert werden Amine bezeichnet, deren Stickstoffatom an eine oder mehrere umfangreiche Gruppen gebunden ist und welche einen kumulativen sterischen Parameter (Taft-Konstante) *E*ₛ von mehr als 1,75 aufweisen.

Die DE 2628376 A1 beschreibt Diamine und Diaminalkanole, welche zur Verwendung in Absorptionsmitteln zur Entfernung von Kohlendioxid aus Gasen geeignet sind.

Die WO 2013/138443 A1 und US 4,894,178 A beschreiben Diaminverbindungen mit zwei sekundären sterisch gehinderten Aminogruppen und deren Verwendung zum Entfernen von CO₂ und/oder H₂S aus Fluidströmen

Der Erfindung liegt die Aufgabe zugrunde, Verbindungen anzugeben, welche eine schnelle Absorption von Kohlendioxid aus Fluidströmen aufweisen, wobei die erforderliche Regenerationsenergie gegenüber Absorptionsmitteln auf der Basis sekundärer und tertiärer Amine nicht wesentlich erhöht ist. Das Absorptionsmittel soll auch für die gleichzeitige Entfernung von H₂S und CO₂ geeignet sein, wobei vorgegebene H₂S-Grenzwerte eingehalten werden müssen, aber eine vollständige Entfernung von CO₂ nicht erforderlich ist.

Die Aufgabe wird gelöst durch die Verwendung einer Verbindung der allgemeinen Formel (I) 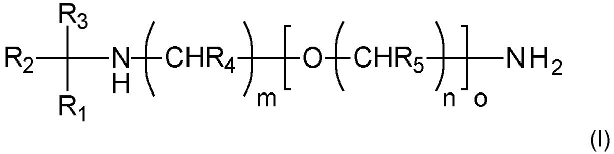 worin R₁, R₂ und R₃ unabhängig voneinander ausgewählt sind unter C₁₋₄-Alkyl und C₁₋₄-Hydroxyalkyl; R₄ in jeder Wiederholungseinheit unabhängig ausgewählt ist unter Wasserstoff, C₁₋₄-Alkyl und C₁₋₄-Hydroxyalkyl; R₅ in jeder Wiederholungseinheit unabhängig ausgewählt ist unter Wasserstoff, C₁₋₄-Alkyl und C₁₋₄-Hydroxyalkyl; m für 2, 3, 4 oder 5 steht; n für 2, 3, 4 oder 5 steht; und o für eine ganze Zahl von 1 bis 10 steht; zum Entfernen von Kohlendioxid und/oder Schwefelwasserstoff aus Fluidströmen. Vorzugsweise stehen R₁, R₂ und R₃ jeweils für Methyl. R₄ steht vorzugsweise für Wasserstoff oder Methyl, insbesondere für Wasserstoff. R₅ steht vorzugsweise für Wasserstoff oder Methyl, insbesondere für Wasserstoff. In einer bevorzugten Ausführungsfrom steht der Rest R₅ am direkt an die primäre Aminogruppe gebundenen Kohlenstoffatom für Wasserstoff. Vorzugsweise steht m für 2, 3 oder 4, insbesondere 2 oder 3, ganz besonders bevorzugt für 2. Vorzugsweise steht n für 2, 3 oder 4, insbesondere 2 oder 3, ganz besonders bevorzugt für 2. Vorzugsweise steht o für 1, 2 oder 3.

Die Erfindung betrifft außerdem Verbindungen der allgemeinen Formel (la) 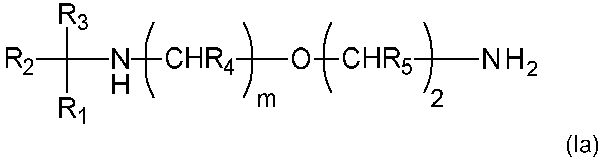 worin R₁, R₂ und R₃ unabhängig voneinander ausgewählt sind unter C₁₋₄-Alkyl und C₁₋₄-Hydroxyalkyl; R₄ jeweils unabhängig ausgewählt ist unter Wasserstoff, C₁₋₄-Alkyl und C₁₋₄-Hydroxyalkyl; R₅ jeweils unabhängig ausgewählt ist unter Wasserstoff, C₁₋₄-Alkyl und C₁₋₄-Hydroxyalkyl; und m für 2, 3, 4 oder 5 steht. In einer bevorzugten Ausführungsform steht der Rest R₅ am direkt an die primäre Aminogruppe gebundenen Kohlenstoffatom für Wasserstoff.

Geeignete Verbindungen der Formel (I) sind 2-(2-tert-Butylaminoethoxy)ethylamin (TBAEEA), 2-(2-(2-tert-Butylaminoethoxy)ethoxy)ethylamin (TBAEEEA), 2-(2-tert-Amylaminoethoxy)ethanamin, 2-(2-(1-Methyl-1-ethylpropylamino)ethoxy) ethanamin und Gemische davon. In einer besonders bevorzugten Ausführungsform ist die Verbindung 2-(2-tert-Butylaminoethoxy)ethylamin (TBAEEA).

Es wird angenommen, dass die primäre Aminogruppe der Verbindung der allgemeinen Formel (I) als Promotor die CO₂-Absorption durch die intermediäre Bildung einer Carbamatstruktur beschleunigt. Die stark sterisch gehinderte sekundäre Aminogruppe wirkt als Basizitäts-Reservoir und bedingt eine hohe zyklische Kapazität.
Die Erfindung betrifft außerdem ein Absorptionsmittel zum Entfernen von Kohlendioxid und/oder Schwefelwasserstoff aus einem Fluidstrom, enthaltend eine wässrige Lösung einer Verbindung der allgemeinen Formel (I).

Die Erfindung betrifft außerdem ein Verfahren zur Entfernung von Kohlendioxid und/oder Schwefelwasserstoff aus einem Fluidstrom, bei dem man den Fluidstrom mit einem Absorptionsmittel in Kontakt bringt, das eine Verbindung der allgemeinen Formel (I) umfasst.

In einer Ausführungsform enthält das Absorptionsmittel wenigstens ein organisches Lösungsmittel. Das organische Lösungsmittel ist bevorzugt ausgewählt unter Sulfolan, Glycolen wie Ethylenglycol, Diethylenglycol, Ethylenglycoldimethylether, Triethylenglycol, Triethylenglycoldimethylether, Di- oder Mono-(C₁₋₄-Alkylether)-monoethylengylcolen und Di- oder Mono-(C₁₋₄-Alkylether)-polyethylengylcolen, N-Methylpyrrolidon, N-Methyl-3-morpholin, N-Formylmorpholin, N-Acetylmorphlin, N,N-Dimethylformamid, N,N-Dimethylimidazolidin-2-on, N-Methylimidazol und Gemischen davon.

In bestimmten Ausführungsformen enthält das Absorptionsmittel wenigstens eine Säure. Die Säure ist geeigneter Weise unter Protonensäuren (Brönstedt-Säuren) ausgewählt. Die Säure ist ausgewählt unter organischen und anorganischen Säuren. Geeignete organische Säuren umfassen beispielsweise Phosphonsäuren, Sulfonsäuren, Carbonsäuren und Aminosäuren. In bestimmten Ausführungsformen ist die Säure eine mehrbasische Säure.

Unter den anorganischen Säuren sind Phosphorsäure und Schwefelsäure bevorzugt.

Unter den Carbonsäuren sind Ameisensäure, Essigsäure, Benzoesäure, Bernsteinsäure und Adipinsäure bevorzugt.

Unter den Sulfonsäuren sind Methansulfonsäure, p-Toluolsulfonsäure und 2-(4-(2-Hydroxyethyl)-1-piperazinyl)-ethansulfonsäure (HEPES) bevorzugt.

Unter den Phosphonsäuren sind 2-Hydroxyphosphonoessigsäure, 2-Phosphonobutan-1,2,4-tricarbonsäure, 1-Hydroxyethan-1,1-diphosphonsäure, Ethylendiamin-tetra-(methylenphosphonsäure), Diethylentriamin-penta(methylenphosphonsäure), Bis(hexamethylen)triamin-penta(methylenphosphonsäure) (HDTMP) und Nitrilo-tris(methylenphosphonsäure) bevorzugt, wovon 1-Hydroxyethan-1,1-diphosphonsäure besonders bevorzugt ist.

In anderen Ausführungsformen ist das Absorptionsmittel frei von Aminocarbonsäuren, Aminosulfonsäuren und Phosphonsäuren.

Im Allgemeinen beträgt die Konzentration von Verbindungen der allgemeinen Formel (I) im Absorptionsmittel 10 bis 60 Gew.-%, bevorzugt 20 bis 50 Gew.-%, besonders bevorzugt 30 bis 50 Gew.-%.

Das Absorptionsmittel kann zusätzlich zur Verbindung der allgemeinen Formel (I) wenigstens ein tertiäres Amin und/oder ein sterisch gehindertes primäres oder sekundäres Amin enthalten.

Das molare Verhältnis der Verbindung der allgemeinen Formel (I) zum tertiären Amin und/oder sterisch gehinderten primären oder sekundären Aminen liegt bevorzugt im Bereich von 0,05 bis 1,0, besonders bevorzugt im Bereich von 0,05 bis 0,7.

Unter einem "tertiären Amin" werden Verbindungen mit wenigstens einer tertiären Aminogruppe verstanden. Das tertiäre Amin enthält vorzugsweise ausschließlich tertiäre Aminogruppen, d. h. es enthält neben wenigstens einer tertiären Aminogruppe keine primären oder sekundären Aminogruppen.

Zu den geeigneten tertiären Aminen zählen insbesondere:

### 1. Tertiäre Alkanolamine wie

Bis(2-hydroxyethyl)-methylamin (Methyldiethanolamin, MDEA), Tris(2-hydroxyethyl)amin (Triethanolamin, TEA), Tributanolamin, 2-Diethylaminoethanol (Diethylethanolamin, DEEA), 2-Dimethylaminoethanol (Dimethylethanolamin, DMEA), 3-Dimethyl-amino-1-propanol (N,N-Dimethylpropanolamin), 3-Diethylamino-1-propanol, 2-Diisopropylaminoethanol (DIEA), N,N-Bis(2-hydroxypropyl)methylamin (Methyldiisopropanolamin, MDIPA);

### 2. Tertiäre Aminoether wie

3-Methoxypropyldimethylamin;

### 3. Tertiäre Polyamine, z. B. bistertiäre Diamine wie

N,N,N',N'-Tetramethylethylendiamin, N,N-Diethyl-N',N'-dimethylethylendiamin, N,N,N',N'-Tetraethylethylendiamin, N,N,N',N'-Tetramethyl-1,3-propandiamin (TMPDA), N,N,N',N'-Tetraethyl-1,3-propandiamin (TEPDA), N,N,N',N'-Tetramethyl-1,6-hexandiamin, N,N-Dimethyl-N',N'-diethylethylendiamin (DMDEEDA), 1-Dimethylamino-2-dimethylaminoethoxyethan (Bis[2-(dimethylamino)ethyl]ether), 1,4-Diazabicyclo[2.2.2]octan (TEDA), Tetramethyl-1,6-hexandiamin;
und Gemische davon.

Tertiäre Alkanolamine, d. h. Amine mit wenigstens einer an das Stickstoffatom gebundenen Hydroxyalkylgruppe, sind im Allgemeinen bevorzugt. Besonders bevorzugt ist Methyldiethanolamin (MDEA).

Unter einer sterischen Hinderung wird die Anwesenheit mindestens eines sekundären oder tertiären Kohlenstoffatoms in unmittelbarer Nachbarschaft zu der sterisch gehinderten Position verstanden. Derartige Amine umfassen neben sterisch gehinderten Aminen auch Verbindungen, die im Stand der Technik als stark sterisch gehinderte Amine bezeichnet werden und einen sterischen Parameter (Taft-Konstante) Eₛ von mehr als 1,75 aufweisen.

Unter einem sekundären Kohlenstoffatom wird ein Kohlenstoffatom, welches außer der Bindung zur sterisch gehinderten Position zwei Kohlenstoff-Kohlenstoff-Bindungen aufweist, verstanden. Unter einem tertiären Kohlenstoffatom wird ein Kohlenstoffatom, welches außer der Bindung zur sterisch gehinderten Position drei Kohlenstoff-Kohlenstoff-Bindungen aufweist, verstanden. Unter einem sekundären Amin wird eine Verbindung mit einem Stickstoffatom, welches mit zwei von Wasserstoff verschiedenen organischen Resten (z. B. Alkyl-Rest, Alkenyl-Rest, Aryl-Rest, Alkylaryl-Rest etc.) substituiert ist, verstanden.

Geeignete sterisch gehinderte primäre oder sekundäre Amine sind beispielsweise 2-(2-tert-Butylaminoethoxy)ethanol (TBAEE), 2-(Isopropylamino)ethanol (IPAE) und 2-Amino-2-methylpropanol (2-AMP).

In anderen Ausführungsformen enthält das Absorptionsmittel wenigstens ein sterisch ungehindertes primäres oder sekundäres Amin. Das sterisch ungehinderte primäre oder sekundäre Amin enthält in seinem Molekül wenigstens eine sterisch ungehinderte primäre oder sekundäre Aminogruppe, d. h. ein Amin-Stickstoffatom, an das nur Wasserstoffatome und primäre Kohlenstoffatome gebunden sind. Sterisch ungehinderte primäre oder sekundäre Amine können als Promotoren die CO₂-Absorption durch intermediäre Bildung einer Carbamatstruktur beschleunigen.

Das sterisch ungehinderte primäre oder sekundäre Amin ist beispielsweise ausgewählt unter
Alkanolaminen, wie Monoethanolamin (MEA), Diethanolamin, (DEA), Ethylaminoethanol, 1-Amino-2-methyl-propan-2-ol, 2-Amino-1-butanol, 2-(2-Aminoethoxy)ethanol und 2-(2-Aminoethoxy)ethanamin,
Polyaminen, wie Hexamethylendiamin, 1,4-Diaminobutan, 1,3-Diaminopropan, 3-(Methylamino)propylamin (MAPA), N-(2-Hydroxyethyl)ethylendiamin, 3-(Dimethylamino)propylamin (DMAPA), 3-(Diethylamino)propylamin, N,N'-Bis(2-hydroxyethyl)ethylendiamin,
5-, 6- oder 7-gliedrigen gesättigten Heterocyclen mit wenigstens einer NH-Gruppe im Ring, die ein oder zwei weitere, unter Stickstoff und Sauerstoff ausgewählte Heteroatome im Ring enthalten können, wie Piperazin, 2-Methylpiperazin, N-Methylpiperazin, N-Ethylpiperazin, N-(2-Hydroxyethyl)piperazin, N-(2-Aminoethyl)piperazin, Homopiperazin, Piperidin und Morpholin.

Besonders bevorzugt sind 5-, 6- oder 7-gliedrige gesättigte Heterocyclen mit wenigstens einer NH-Gruppe im Ring, die ein oder zwei weitere, unter Stickstoff und Sauerstoff ausgewählte Heteroatome im Ring enthalten können. Ganz besonders bevorzugt ist Piperazin.

Das molare Verhältnis der Verbindung der allgemeinen Formel (I) zum sterisch ungehinderten primären oder sekundären Amin liegt bevorzugt im Bereich von 1,0 bis 20, besonders bevorzugt im Bereich von 1,5 bis 15.

Das Absorptionsmittel kann auch Additive, wie Korrosionsinhibitoren, Enzyme, etc. enthalten. Im Allgemeinen liegt die Menge an derartigen Additiven im Bereich von etwa 0,01 bis 3 Gew.-% des Absorptionsmittels.

Das erfindungsgemäße Absorptionsmittel bzw. Verfahren ist geeignet zur Behandlung von Fluiden aller Art. Fluide sind einerseits Gase, wie Erdgas, Synthesegas, Koksofengas, Spaltgas, Kohlevergasungsgas, Kreisgas, Deponiegase und Verbrennungsgase, und andererseits mit dem Absorptionsmittel im Wesentlichen nicht mischbare Flüssigkeiten, wie Flüssiggaskraftstoff (LPG, Liquefied Petroleum Gas) oder verflüssigtes Erdgas (NGL, Natural Gas Liquids). In einer Ausführungsform ist der Fluidstrom ein Rauchgasstrom, z. B. aus Verbrennungsanlagen, Produktionsgasen, Synthesegasen oder auch Raumluft. Diese Gase entstehen u. a. bei Kraftwerken, Kraftfahrzeugen, Produktionsstätten, Ammoniakproduktion, Epoxidherstellung, Zementproduktion, Keramikindustrie, Kokereien, Metallverhüttung, Stahlindustrie, Treibmittelexposition und klimatisierten Arbeits- und Wohnbereichen. Weitere CO₂-haltige Fluidströme sind Gärgase aus der Methanogenese von Biomassen, Faulgase aus der aeroben und/oder anaeroben Kompostierung von Biomassen, Verbrennungsgase, tierische Verdauungsgase in der Massentierhaltung und CO₂-haltige Raumluft in der Gebäude- und Fahrzeugklimatechnik.

Der Fluidstrom enthält Kohlendioxid und/oder Schwefelwasserstoff; er kann daneben weitere saure Gase, wie COS und Mercaptane, enthalten. Außerdem können auch SO₃, SO₂, CS₂ und HCN entfernt werden.

Die Verbindungen der allgemeinen Formel (I) sind vor allem zur Verwendung in Verfahren bzw. Absorptionsmitteln zur Behandlung von kohlenwasserstoffhaltigen Fluidströmen geeignet. Die enthaltenen Kohlenwasserstoffe sind z. B. aliphatische Kohlenwasserstoffe, wie C₁-C₄-Kohlenwasserstoffe, wie Methan, ungesättigte Kohlenwasserstoffe, wie Ethylen oder Propylen, oder aromatische Kohlenwasserstoffe wie Benzol, Toluol oder Xylol. Insbesondere ist das erfindungsgemäße Verfahren zur Behandlung eines Erdgasstroms geeignet. Das erfindungsgemäße Verfahren bzw. Absorptionsmittel ist besonders zur Entfernung von CO₂ geeignet.

In bevorzugten Ausführungsformen liegt im Fluidstrom ein Kohlendioxid-Partialdruck im Bereich von 0,01 bis weniger als 3,0 bar, insbesondere 0,03 bis weniger als 3,0 bar vor. Die angegebenen Partialdrücke beziehen sich auf den Fluidstrom beim erstmaligen Kontakt mit dem Absorptionsmittel im Absorptionsschritt.

Der Schwefelwasserstoff-Partialdruck im Fluidstrom beträgt üblicherweise mindestens 2,5 mbar. In bevorzugten Ausführungsformen liegt im Fluidstrom ein Schwefelwasserstoff-Partialdruck von mindestens 0,1 bar, insbesondere mindestens 1 bar, und ein Kohlendioxid-Partialdruck von mindestens 0,2 bar, insbesondere mindestens 1 bar, vor. Die angegebenen Partialdrücke beziehen sich auf den Fluidstrom beim erstmaligen Kontakt mit dem Absorptionsmittel im Absorptionsschritt. Im erfindungsgemäßen Verfahren wird der Fluidstrom in einem Absorptionsschritt in einem Absorber in Kontakt mit dem Absorptionsmittel gebracht, wodurch Kohlendioxid und/oder Schwefelwasserstoff zumindest teilweise ausgewaschen werden. Man erhält einen CO₂- bzw. H₂S- abgereicherten Fluidstrom und ein CO₂- bzw. H₂S-beladenes Absorptionsmittel.

Als Absorber fungiert eine in üblichen Gaswäscheverfahren eingesetzte Waschvorrichtung. Geeignete Waschvorrichtungen sind beispielsweise Füllkörper-, Packungs- und Bodenkolonnen, Membrankontaktoren, Radialstromwäscher, Strahlwäscher, Venturi-Wäscher und Rotations-Sprühwäscher, bevorzugt Packungs-, Füllkörper- und Bodenkolonnen, besonders bevorzugt Boden- und Füllkörperkolonnen. Die Behandlung des Fluidstroms mit dem Absorptionsmittel erfolgt dabei bevorzugt in einer Kolonne im Gegenstrom. Das Fluid wird dabei im Allgemeinen in den unteren Bereich und das Absorptionsmittel in den oberen Bereich der Kolonne eingespeist. In Bodenkolonnen sind Sieb-, Glocken- oder Ventilböden eingebaut, über welche die Flüssigkeit strömt. Füllkörperkolonnen können mit unterschiedlichen Formkörpern gefüllt werden. Wärme- und Stoffaustausch werden durch die Vergrößerung der Oberfläche aufgrund der meist etwa 25 bis 80 mm großen Formkörper verbessert. Bekannte Beispiele sind der Raschig-Ring (ein Hohlzylinder), Pall-Ring, Hiflow-Ring, Intalox-Sattel und dergleichen. Die Füllkörper können geordnet, aber auch regellos (als Schüttung) in die Kolonne eingebracht werden. Als Materialien kommen in Frage Glas, Keramik, Metall und Kunststoffe. Strukturierte Packungen sind eine Weiterentwicklung der geordneten Füllkörper. Sie weisen eine regelmäßig geformte Struktur auf. Dadurch ist es bei Packungen möglich, Druckverluste bei der Gasströmung zu reduzieren. Es gibt verschiedene Ausführungen von Packungen z. B. Gewebe- oder Blechpackungen. Als Material können Metall, Kunststoff, Glas und Keramik eingesetzt werden.

Die Temperatur des Absorptionsmittels beträgt im Absorptionsschritt im Allgemeinen etwa 30 bis 100°C, bei Verwendung einer Kolonne beispielsweise 30 bis 70°C am Kopf der Kolonne und 50 bis 100°C am Boden der Kolonne. Der Gesamtdruck beträgt im Absorptionsschritt im Allgemeinen etwa 1 bis 180 bar, bevorzugt etwa 1 bis 100 bar.

Das erfindungsgemäße Verfahren kann einen oder mehrere, z. B. zwei, aufeinander folgende Absorptionsschritte umfassen. Die Absorption kann in mehreren aufeinander folgenden Teilschritten durchgeführt werden, wobei das die sauren Gasbestandteile enthaltende Rohgas in jedem der Teilschritte mit jeweils einem Teilstrom des Absorptionsmittels in Kontakt gebracht wird. Das Absorptionsmittel, mit dem das Roh-Rohgas in Kontakt gebracht wird, kann bereits teilweise mit sauren Gasen beladen sein, d. h. es kann sich beispielsweise um ein Absorptionsmittel, das aus einem nachfolgenden Absorptionsschritt in den ersten Absorptionsschritt zurückgeführt wurde, oder um teilregeneriertes Absorptionsmittel handeln. Bezüglich der Durchführung der zweistufigen Absorption wird Bezug genommen auf die Druckschriften EP 0 159 495, EP 0 190 434, EP 0 359 991 und WO 00100271.

Das Verfahren umfasst bevorzugt einen Regenerationsschritt, bei dem man das CO₂- und H₂S-beladene Absorptionsmittel regeneriert. Im Regenerationsschritt werden aus dem CO₂- und H₂S-beladenen Absorptionsmittel CO₂ und H₂S und gegebenenfalls weitere saure Gasbestandteile freigesetzt, wobei ein regeneriertes Absorptionsmittel erhalten wird. Vorzugsweise wird das regenerierte Absorptionsmittel anschließend in den Absorptionsschritt zurückgeführt. In der Regel umfasst der Regenerationsschritt wenigstens eine der Maßnahmen Erwärmung, Entspannung und Strippen mit einem inerten Fluid.

Der Regenerationsschritt umfasst bevorzugt eine Erwärmung des mit den sauren Gasbestandteilen beladenen Absorptionsmittels. Die absorbierten Sauergase werden dabei mittels des durch Erhitzens der Lösung gewonnenen Wasserdampfes abgestrippt. Anstelle des Dampfes kann auch ein inertes Fluid, wie Stickstoff, verwendet werden. Der absolute Druck im Desorber liegt normalerweise bei 0,1 bis 3,5 bar, bevorzugt 1,0 bis 2,5 bar. Die Temperatur liegt normalerweise bei 50 °C bis 170 °C, bevorzugt bei 80 °C bis 130 °C, wobei die Temperatur natürlich vom Druck abhängig ist.

Der Regenerationsschritt kann alternativ oder zusätzlich eine Druckentspannung umfassen. Diese beinhaltet mindestens eine Druckentspannung des beladenen Absorptionsmittels von einem hohen Druck, wie er bei der Durchführung des Absorptionsschritts herrscht, auf einen niedrigeren Druck. Die Druckentspannung kann beispielsweise mittels eines Drosselventils und/oder einer Entspannungsturbine geschehen. Die Regeneration mit einer Entspannungsstufe ist beispielsweise beschrieben in den Druckschriften US 4,537, 753 und US 4,553, 984.

Die Freisetzung der sauren Gasbestandteile im Regenerationsschritt kann beispielsweise in einer Entspannungskolonne, z. B. einem senkrecht oder waagerecht eingebauten Flash-Behälter oder einer Gegenstromkolonne mit Einbauten, erfolgen.

Bei der Regenerationskolonne kann es sich ebenfalls um eine Füllkörper-, Packungs- oder Bodenkolonne handeln. Die Regenerationskolonne weist am Sumpf einen Aufheizer auf, z. B. einen Aufkocher, Naturumlaufverdampfer, Zwangsumlaufverdampfer, oder Zwangsumlaufentspannungsverdampfer. Am Kopf weist die Regenerationskolonne einen Auslass für die freigesetzten Sauergase auf. Mitgeführte Absorptionsmitteldämpfe werden in einem Kondensator kondensiert und in die Kolonne zurückgeführt.

Es können mehrere Entspannungskolonnen hintereinander geschaltet werden, in denen bei unterschiedlichen Drücken regeneriert wird. Beispielsweise kann in einer Vorentspannungskolonne bei hohem Druck, der typischerweise etwa 1,5 bar oberhalb des Partialdrucks der sauren Gasbestandteile im Absorptionsschritt liegt, und in einer Hauptentspannungskolonne bei niedrigem Druck, beispielsweise 1 bis 2 bar absolut, regeneriert werden. Die Regeneration mit zwei oder mehr Entspannungsstufen ist beschrieben in den Druckschriften US 4,537, 753, US 4,553, 984, EP 0 159 495, EP 0 202 600, EP 0 190 434 und EP 0 121 109.

Die Erfindung wird anhand der beigefügten Zeichnungen und der nachfolgenden Beispiele näher veranschaulicht.
Fig. 1 ist eine schematische Darstellung einer zur Durchführung des erfindungsgemäßen Verfahrens geeigneten Anlage.
Fig. 2 ist eine schematische Darstellung einer zur Bestimmung der relativen CO₂-Absorptionsgeschwindigkeiten von Absorptionsmitteln verwendeten Doppelrührzellenanordnung.
Fig. 3 zeigt den pH-Wert von wässrigen Lösungen von MDEA bzw. MDEA + TBAEEA in Abhängigkeit von der Temperatur.
Fig. 4 zeigt die zyklische Kapazität von wässrigen MDEA-Lösungen mit Piperazin, TBAEE und TBAEEA.

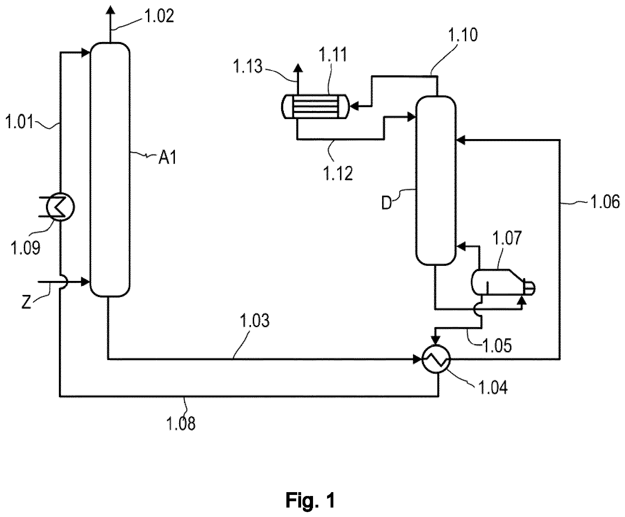

Gemäß Fig. 1 wird über die Zuleitung Z ein geeignet vorbehandeltes, Schwefelwasserstoff und/oder Kohlendioxid enthaltendes Gas in einem Absorber A1 mit regeneriertem Absorptionsmittel, das über die Absorptionsmittelleitung 1.01 zugeführt wird, im Gegenstrom in Kontakt gebracht. Das Absorptionsmittel entfernt Schwefelwasserstoff und/oder Kohlendioxid durch Absorption aus dem Gas; dabei wird über die Abgasleitung 1.02 ein an Schwefelwasserstoff und/oder Kohlendioxid abgereiabgereichertes Reingas gewonnen.

Über die Absorptionsmittelleitung 1.03, den Wärmetauscher 1.04, in dem das CO₂- und/oder H₂S-beladene Absorptionsmittel mit der Wärme des über die Absorptionsmittelleitung 1.05 geführten, regenerierten Absorptionsmittels aufgeheizt wird, und die Absorptionsmittelleitung 1.06 wird das mit CO₂- und/oder H₂S-beladene Absorptionsmittel der Desorptionskolonne D zugeleitet und regeneriert. Aus dem unteren Teil der Desorptionskolonne D wird das Absorptionsmittel in den Aufkocher 1.07 geführt, wo es erhitzt wird. Der hauptsächlich wasserhaltige Dampf wird in die Desorptionskolonne D zurückgeführt, während das regenerierte Absorptionsmittel über die Absorptionsmittelleitung 1.05, den Wärmetauscher 1.04, in dem das regenerierte Absorptionsmittel das CO₂- und/oder H₂S-beladene Absorptionsmittel aufheizt und selbst dabei abkühlt, die Absorptionsmittelleitung 1.08, den Kühler 1.09 und die Absorptionsmittelleitung 1.01 dem Absorber A1 wieder zugeführt wird. Anstelle des gezeigten Aufkochers können auch andere Wärmetauschertypen zur Erzeugung des Strippdampfes eingesetzt werden, wie ein Naturumlaufverdampfer, Zwangsumlaufverdampfer, oder Zwangsumlaufentspannungsverdampfer. Bei diesen Verdampfertypen wird ein gemischtphasiger Strom aus regeneriertem Absorptionsmittel und Strippdampf in den Sumpf der Desorptionskolonne zurückgefahren, wo die Phasentrennung zwischen dem Dampf und dem Absorptionsmittel statt. Das regenerierte Absorptionsmittel zum Wärmetauscher 1.04 wird entweder aus dem Umlaufstrom vom Sumpf der Desorptionskolonne zum Verdampfer abgezogen, oder über eine separate Leitung direkt aus dem Sumpf der Desorptionskolonne zum Wärmetauscher 1.04 geführt.

Das in der Desorptionskolonne D freigesetzte CO₂- und/oder H₂S-haltige Gas verlässt die Desorptionskolonne D über die Abgasleitung 1.10. Es wird in einen Kondensator mit integrierter Phasentrennung 1.11 geführt, wo es von mitgeführtem Absorptionsmitteldampf getrennt wird. Kondensation und Phasentrennung können auch getrennt voneinander vorliegen. Anschließend wird eine hauptsächlich aus Wasser bestehende Flüssigkeit über die Absorptionsmittelleitung 1.12 in den oberen Bereich der Desorptionskolonne D geführt, und ein CO₂- und/oder H₂S-haltiges Gas über die Gasleitung 1.13 ausgeführt.

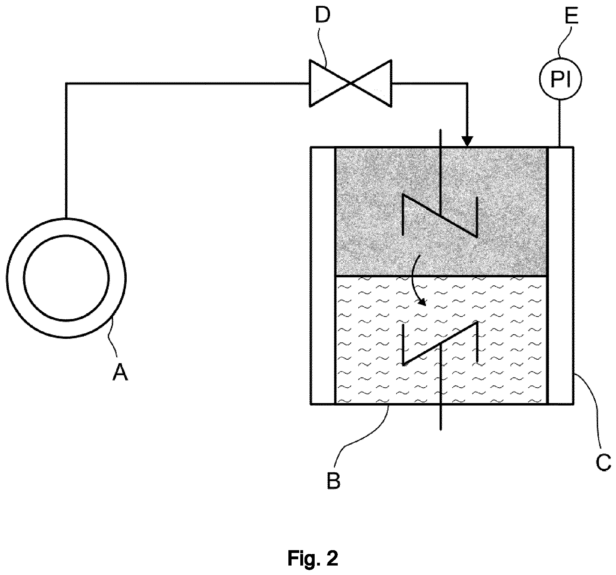

In Fig. 2 werden die folgenden Referenzzeichen verwendet: A = CO₂-Speicherbehälter, B = Doppelrührzelle, C = Temperaturregler, D = Dosierventil, E = Druckmessgerät. Gemäß Fig. 2 befindet sich im unteren Teil der Doppelrührzelle B eine Flüssigphase des zu prüfenden Absorptionsmittels, welche mit der darüber liegenden Gasphase über eine Phasengrenze in Kontakt steht. Die Flüssig- bzw. Gasphase können jeweils mit einem Rührer durchmischt werden. Die Doppelrührzelle B ist über ein Dosierventil D mit dem CO₂-Speicherbehälter A verbunden. Der in der Doppelrührzelle B herrschende Druck kann mittels des Druckmessgeräts E bestimmt werden. Bei der Messung wird der Volumenstrom des Kohlendioxids aufgezeichnet, wobei der Volumenstrom so eingestellt ist, dass ein konstanter Druck in Doppelrührzelle B herrscht.

### Beispiele

Es werden folgende Abkürzungen verwendet:
DRZ: Doppelrührzelle
HPCy₂: Dicyclohexylphosphin
MDEA: Methyldiethanolamin
MeOH: Methanol
MTBE: Methyl-tert-butylether
TBAEE: 2-(2-tert-Butylaminoethoxy)ethanol
TBAEEA: 2-(2-tert-Butylaminoethoxy)ethylamin
THF: Tetrahydrofuran

### Beispiel 1

### Synthese von 2-(2-tert-Butylaminoethoxy)ethylamin (TBAEEA)

### A) Synthese von Katalysatorkomplex A

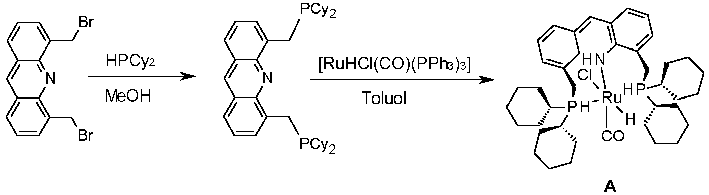

### A1) Synthese von 4,5-Bis(dicyclohexylphosphinomethyl)acridin

Eine Lösung aus 4,5-Bis(bromomethyl)acridin (5,2 g, 14,2 mmol) und Dicyclohexylphosphin (8,18 g, 36,8 mmol) in 65 mL wasserfreiem, entgastem Methanol wurde 66 h unter einer Argon-Atmosphäre auf 50 °C erhitzt. Nach Abkühlen auf Raumtemperatur wurde Triethylamin (5,72 g, 56,7 mmol) zugegeben und 1 h gerührt. Verdampfen des Lösungsmittels ergab einen gelb-weißen Feststoff in rotem Öl. Extraktion mittels 3 x 40 mL Methyl-tert-butylether (MTBE) und Konzentration des Filt-Filtrats ergaben ein rotbraunes Öl (¹H NMR: Mischung aus Produkt und HPCy₂). Das Öl wurde in wenig warmem MTBE aufgenommen und eisgekühltes Methanol zugegeben, was die Ausfällung eines gelben, mikrokristallinen Feststoffes bewirkte. Isolierung und Trocknung im Vakuum ergab luftempfindliches 4,5-Bis(dicyclohexylphosphinomethyl)acridin (2,74 g, 33%) als gelbes Pulver.

### A2) Synthese des Katalysatorkomplexes A

4,5-Bis(dicyclohexylphosphinomethyl)acridin (1855 mg, 3,1 mmol) und [RuHCl(CO)(PPh₃)₃]² (2678 mg, 2,81 mmol) wurden für 2 h in 80 mL entgastem Toluol auf 70 °C erhitzt. Die resultierende dunkelbraune Lösung wurde zur Trockene eingeengt, der Rückstand in 3 x 20 mL Hexan suspendiert und durch Filtration isoliert. Trocknen im Vakuum ergab den Katalysatorkomplex A (1603 mg, 75%) als orangebraunes Pulver.

### B) Synthese von 2-(2-tert-Butylaminoethoxy)ethylamin (TBAEEA)

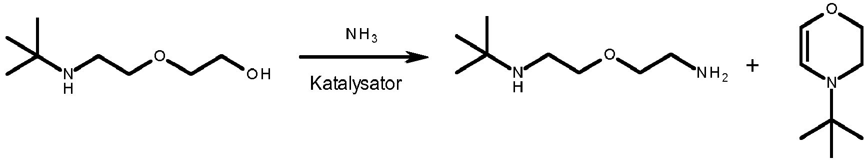

Katalysatorkomplex A (38,3 mg), THF (50 mL) und tert-Butylaminoethoxyethanol wurden unter einer Argon-Atmosphäre in einem 160 mL Parr-Autoklaven (Edelstahl V4A) mit magnetisch gekoppeltem Schrägblattrührer (Rührgeschwindigkeit: 200-500 Umdrehungen/Minute) vorgelegt. Ammoniak (20,5 g) wurde bei Raumtemperatur vorkondensiert und zudosiert. Der Stahlautoklav wurde auf 180 °C elektrisch aufgeheizt und für 24 h unter Rühren (500 Umdrehungen/Minute) erhitzt (Innentemperaturmessung). Nach Erhitzen der Reaktionsmischung im Autoklaven auf 180 °C entwickelte sich ein Eigendruck von 89 bar. Nach dem Abkühlen auf Raumtemperatur, Entspannung des Autoklaven und Ausgasen des Ammoniaks bei Normaldruck wurde die Reaktionsmischung mittels GC (Säule "Rtx®-5 Amine", Länge 30 m, Innendurchmesser 0,32 mm, d_{f} 1,5 µm, 60 °C - 4 °C/min - 280 °C) analysiert. Bei quantitativem Umsatz sind 90% des gewünschten 2-(2-tert-Butylamino-ethoxy)ethylamins gemäß GC-FI%-Auswertung entstanden. Hauptnebenkomponente ist mit 6 % das abgebildete zyklisierte Morpholinderivat. Das Produkt wurde durch Des-Destillation gereinigt (Übergangstemperatur 70 °C bei 0.5 mbar).

### Beispiel 2

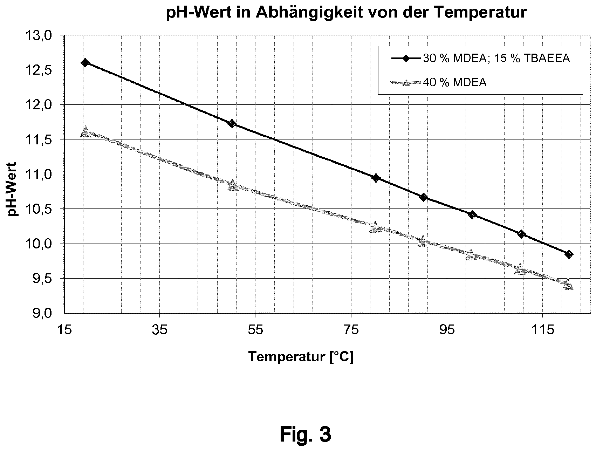

Für Mischungen bestehend aus 40 Gew.-% MDEA und 60 Gew.-% Wasser (2-1) bzw. 30 Gew.-% MDEA, 15 % Gew.-% TBAEEA und 55 Gew.-% Wasser (2-2) wurde die Temperaturabhängigkeit des pH-Wertes bestimmt. Es wurde eine Druckapparatur verwendet, in der der pH-Wert bis 120 °C gemessen werden kann. Die Ergebnisse sind in Fig. 3 dargestellt. Die Mischung mit TBAEEA (2-2) zeigt bei 20 °C einen deutlich höheren pH-Wert als die Mischung mit MDEA (2-1). Der pH-Wert ist ein Maß dafür, wie gut CO₂ oder H₂S gebunden werden können. Je höher der pH-Wert der Lösung ist, desto mehr CO₂ oder auch H₂S kann gebunden werden. D.h. bei niedrigen Temperaturen, wie sie typischerweise in Absorbern herrschen, ist ein hoher pH-Wert vorteilhaft. Insgesamt zeigt die Mischung TBAEEA + MDEA (2-2) eine stärkere Temperaturabhängigkeit als das Referenzbeispiel mit MDEA (2-1). Für die TBAEEA und MDEA enthaltende Lösung (2-2) beträgt der Gradient -0,027 pH-Einheiten / K, für die MDEA-Lösung (2-1) hingegen nur -0,022 pH-Einheiten / K. Für die Regeneration ist eine möglichst hohe pH-Differenz zwischen höheren und niedrigeren Temperaturen vorteilhaft, da die absorbierten sauren Komponenten bei höheren Temperaturen und dementsprechend niedrigeren pH-Werten mit weniger Energieaufwand wieder abgegeben werden.

### Beispiel 3

In einer Doppelrührzelle (DRZ) gemäß Fig. 2 wurden die relativen CO₂-Absorptionsgeschwindigkeiten von wässrigen Absorptionsmitteln gemessen.

Die Doppelrührzelle hatte einen Innendurchmesser von 85 mm und ein Volumen von 509 mL. Die Temperatur der Zelle wurde während den Messungen auf 40 °C gehalten. Um die Gas- und Flüssigphasen zu durchmischen, umfasste die Zelle gemäß Fig. 2 zwei Rührer. Vor Beginn der Messung wurde die Doppelrührzelle evakuiert. Ein definiertes Volumen an entgastem Absorptionsmittel wurde in die Doppelrührzelle gegeben und die Temperatur auf 40 °C reguliert. Die Rührer wurden bereits während des Aufheizens des unbeladenen Absorptionsmittels eingeschaltet. Die Drehzahl der Rührer wurde so gewählt, dass sich eine planare Phasengrenzfläche zwischen der Flüssig- und der Gasphase ausbildete. Eine Wellenentwicklung an der Phasengrenzfläche muss vermieden werden, da sonst keine definierte Phasengrenzfläche bestehen würde. Nachdem die gewünschte experimentelle Temperatur erreicht war, wurde Kohlendioxid mittels eines Dosierventils in den Reaktor eingeführt. Der Volumenstrom wurde so kontrolliert, dass während des gesamten Versuchs ein konstanter Druck von 100 mbar abs herrschte. Mit zunehmender Versuchszeit nahm der Volumenstrom ab, da das Absorptionsmedium über die Zeit gesätgesättigt wurde und die Absorptionsgeschwindigkeit abnahm. Der Volumenstrom wurde über die gesamte Zeit aufgezeichnet. Zu Versuchsbeginn betrug die Kohlendioxid-Flussrate etwa 4 NL/h. Der Versuch wurde beendet, sobald die Kohlendioxid-Flussrate weniger als 0,02 NL/h betrug. Das Absorptionsmedium war am Ende des Versuchs in einem Gleichgewichtszustand.

Es wurden folgende Absorptionsmittel untersucht:
3-1) wässrige Lösung von MDEA (41 Gew.-%)
3-2) wässrige Lösung von MDEA (30 Gew.-%) + TBAEE (15 Gew.-%)
3-3) wässrige Lösung von MDEA (30 Gew.-%) + TBAEEA (15 Gew.-%)

Die Absorptionsgeschwindigkeit wurde bei 20 bzw. 50 % der am Versuchsende erreichten Beladung (GGW) bestimmt. Die Werte wurden auf die Absorptionsrate des Absorptionsmittels 3-1 bei 20 % bzw. 50 % GGW normiert. Die Ergebnisse sind in der nachstehenden Tabelle angegeben:

| Beispiel | System | Relative Absorptionsrate bei 20 % GGW** | Relative Absorptionsrate bei 50 % GGW** |
|---|---|---|---|
| 3-1* | MDEA (41 Gew.-%) | 100 % | 100 % |
| 3-2* | MDEA (30 Gew.-%) + TBAEE (15 Gew.-%) | 136 % | 128 % |
| 3-3 | MDEA (30 Gew.-%) + TBAEEA (15 Gew.-%) | 355 % | 309 % |

| | | | |
|---|---|---|---|
| * Vergleichsbeispiel ** bezogen auf Beispiel 3-1 | | | |

Es ist erkennbar, dass im erfindungsgemäßen Beispiel 3-3 die Absorptionsgeschwindigkeit deutlich höher ist als in den Vergleichsbeispielen 3-1 und 3-2, unabhängig von der jeweiligen CO₂-Beladung.

### Beispiel 4

Zur Abschätzung der zyklischen Kapazität wurden ein Beladungsversuch und ein anschließender Strippversuch für folgende wässrige Absorptionsmittel durchgeführt:
4-1) 30 Gew.-% MDEA + 8 Gew.-% Piperazin
4-2) 30 Gew.-% MDEA + 15 Gew.-% TBAEE
4-3) 30 Gew.-% MDEA + 15 Gew.-% TBAEEA

Als Apparatur wurde ein thermostatisierter Glaszylinder mit aufgesetztem Rückflusskühler verwendet. Der Rückflusskühler wurde bei einer Temperatur von ca. 5 °C betrieben und verhinderte, dass Wasser und Amin während der Beladung bzw. der Strippung ausgetragen wurde.

Bei 40 °C wurden jeweils 100 mL des Absorptionsmittels in den Glaszylinder eingefüllt. Über eine Fritte am unteren Ende des Glaszylinders wurden für ca. 4 h 8 NL/h reines CO₂ in die Absorptionslösung eingeperlt. Am Ende des Versuchs wurde die Beladung an CO₂ im Absorptionsmittel mittels Messung des Gehalts an Total Inorganic Carbon (TIC) bestimmt.

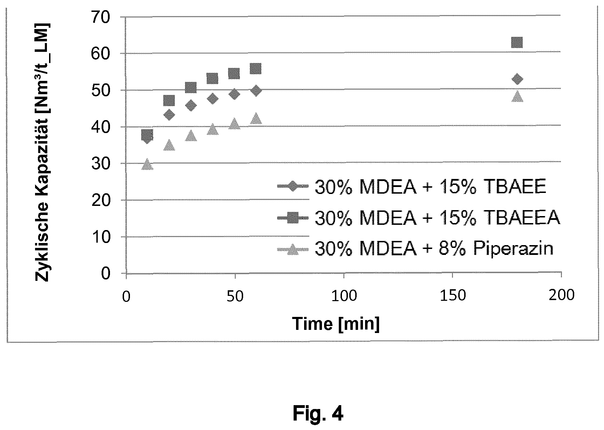

Die beladenen Lösungen wurden in einer identisch aufgebauten Apparatur bei 80 °C mit Stickstoff (8 NL/h) gestrippt. Über 60 min wurden alle 10 min Proben des Absorptionsmittels entnommen und auf den CO₂-Gehalt analysiert. Die Strippversuche wurden weitere 2 h fortgesetzt und abschließend die Beladung an CO₂ im Absorptionsmittel bestimmt (Gesamtstrippdauer: 180 min). Aus der Differenz der am Ende des Beladungsversuchs erreichten CO₂-Beladung und der in Abhängigkeit der Strippdauer ermittelten CO₂-Beladung ergeben sich die zyklischen Kapazitäten der drei Absorptionsmittel. Die Ergebnisse sind in Figur 4 dargestellt. Es zeigt sich dabei, dass die Mischung mit TBAEEA (4-3) die höchste zyklische Kapazität aufweist, unabhängig von der Strippdauer.

### Beispiel 5

Es wurde die Flüchtigkeit folgender Absorptionsmittel ermittelt:
5-1) 41 Gew.-% MDEA
5-2) 30 Gew.-% MDEA + 15 Gew.-% TBAEE
5-3) 30 Gew.-% MDEA + 15 Gew.-% TBAEEA

Eine möglichst niedrige Flüchtigkeit ist vorteilhaft, um einen möglichst geringen Austrag von Aminen mit dem gereinigten Gas oder mit dem abgetrennten Sauergas aus der Absorptionsanlage zu gewährleisten.

Es wurde eine Apparatur gemäß der in Beispiel 4 beschriebenen Beladungs- bzw. Strippapparatur verwendet. Der Glaszylinder wurde auf eine Temperatur von 50 °C erhitzt und dort gehalten und jeweils 100 mL des Absorptionsmittels eingefüllt. Über eine Fritte am unteren Ende des Glaszylinders wurden für ca. 8 h 20 NL/h reines CO₂ in die Absorptionslösung eingeperlt. Die auskondensierten Flüssigkeiten wurden im Gegensatz zu Beispiel 4 nicht zurück in den Glaszylinder geleitet, sondern getrennt gesammelt und nach Versuchsende auf ihre Zusammensetzung analysiert. Die Ergebnisse sind in folgenfolgender Tabelle dargestellt:

| Beispiel | System | Kondensatmenge [g] | Zusammensetzung Kondensat | | | |
|---|---|---|---|---|---|---|
| | | | Wasser [g/ 100 g] | MDEA [g/ 100 g] | TBAEE [g / 100 g] | TBAEEA [g / 100 g] |
| 5-1* | MDEA | 15,811 | 99,1 | 0,37 | - | - |
| 5-2* | MDEA + TBAEE | 17,284 | 99,2 | 0,39 | 0,37 | - |
| 5-3 | MDEA + TBAEEA | 16,949 | 99,2 | 0,42 | - | 0,27 |

| | | | | | | |
|---|---|---|---|---|---|---|
| * Vergleichsbeispiel | | | | | | |

Es ist erkennbar, dass im Beispiel 5-3 die Flüchtigkeit der erfindungsgemäßen Verbindung TBAEEA deutlich niedriger ist als die der Verbindungen in den Vergleichsbeispielen 5-1 und 5-2.

## Patentansprüche

1. Verwendung einer Verbindung der allgemeinen Formel (I) 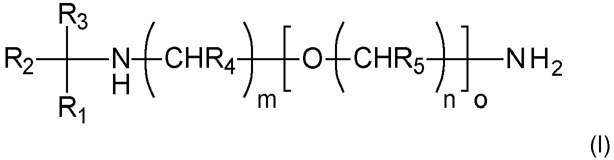
worin R₁, R₂ und R₃ unabhängig voneinander ausgewählt sind unter C₁₋₄-Alkyl und C₁₋₄-Hydroxyalkyl; R₄ jeweils unabhängig ausgewählt ist unter Wasserstoff, C₁₋₄-Alkyl und C₁₋₄-Hydroxyalkyl; R₅ jeweils unabhängig ausgewählt ist unter Wasserstoff, C₁₋₄-Alkyl und C₁₋₄-Hydroxyalkyl; m für 2, 3, 4 oder 5 steht; n für 2, 3, 4 oder 5 steht; und o für eine ganze Zahl von 1 bis 10 steht;
zum Entfernen von Kohlendioxid und/oder Schwefelwasserstoff aus Fluidströmen.

2. Absorptionsmittel zum Entfernen von Kohlendioxid und/oder Schwefelwasserstoff aus Fluidströmen, enthaltend eine wässrige Lösung einer Verbindung der allgemeinen Formel I 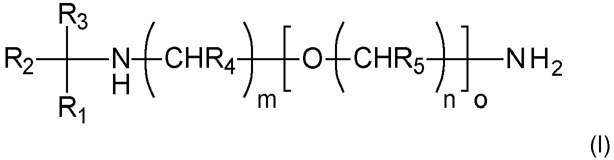 worin R₁, R₂ und R₃ unabhängig voneinander ausgewählt sind unter C₁₋₄-Alkyl und C₁₋₄-Hydroxyalkyl; R₄ jeweils unabhängig ausgewählt ist unter Wasserstoff, C₁₋₄-Alkyl und C₁₋₄-Hydroxyalkyl; R₅ jeweils unabhängig ausgewählt ist unter Wasserstoff, C₁₋₄-Alkyl und C₁₋₄-Hydroxyalkyl; m für 2, 3, 4 oder 5 steht; n für 2, 3, 4 oder 5 steht; und o für eine ganze Zahl von 1 bis 10 steht.

3. Absorptionsmittel nach Anspruch 2, wobei der Rest R₅ am direkt an die primäre Aminogruppe gebundenen Kohlenstoffatom für Wasserstoff steht.

4. Absorptionsmittel nach einem der Ansprüche 2 oder 3, wobei das Absorptionsmittel wenigstens ein organisches Lösungsmittel umfasst.

5. Absorptionsmittel nach einem der Ansprüche 2 bis 4, wobei das Absorptionsmittel wenigstens eine Säure umfasst.

6. Absorptionsmittel nach einem der Ansprüche 2 bis 5, wobei die Konzentration der Verbindung der Formel (I) im Absorptionsmittel 10 bis 60 Gew.-% beträgt.

7. Absorptionsmittel nach einem der Ansprüche 2 bis 6, außerdem enthaltend wenigstens ein tertiäres Amin und/oder ein sterisch gehindertes primäres oder sekundäres Amin.

8. Absorptionsmittel nach Anspruch 7, wobei das tertiäre Amin Methyldiethanolamin ist.

9. Verfahren zum Entfernen von Kohlendioxid und/oder Schwefelwasserstoff aus Fluidströmen, wobei man ein Absorptionsmittel der Ansprüche 2 bis 8 in Kontakt mit einem Fluidstrom bringt.

10. Verfahren nach Anspruch 9, wobei der Fluidstrom Kohlenwasserstoffe enthält.

11. Verfahren nach einem der Ansprüche 9 oder 10, wobei der Fluidstrom einen Gesamtdruck von mindestens 3,0 bar aufweist.

12. Verfahren nach einem der Ansprüche 9 bis 11, umfassend einen Regenerationsschritt, wobei der Regenerationsschritt wenigstens eine der Maßnahmen Erwärmung, Entspannung und Strippen mit einem inerten Fluid umfasst.

13. Verbindung der allgemeinen Formel (la) 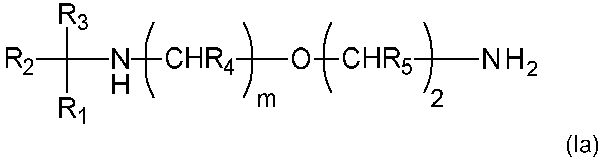 worin R₁, R₂ und R₃ unabhängig voneinander ausgewählt sind unter C₁₋₄-Alkyl und C₁₋₄-Hydroxyalkyl; R₄ jeweils unabhängig ausgewählt ist unter Wasserstoff, C₁₋₄-Alkyl und C₁₋₄-Hydroxyalkyl; R₅ jeweils unabhängig ausgewählt ist unter Wasserstoff, C₁₋₄-Alkyl und C₁₋₄-Hydroxyalkyl; und m für 2, 3, 4 oder 5 steht.

14. Verbindung nach Anspruch 13 mit der Maßgabe, dass der Rest R₅ am direkt an die primäre Aminogruppe gebundenen Kohlenstoffatom für Wasserstoff steht.

15. Verbindung nach einem der Ansprüche 13 oder 14, nämlich 2-(2-tert-Butylaminoethoxy)ethylamin.

## Claims

1. Use of compound of the general formula (I) 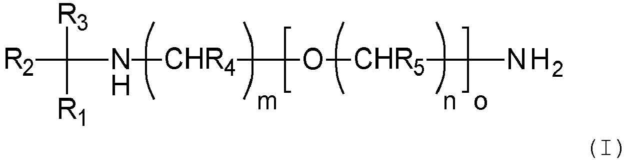
in which R₁, R₂ and R₃ are each independently selected from C₁₋₄-alkyl and C₁₋₄-hydroxyalkyl; each R₄ is independently selected from hydrogen, C₁₋₄-alkyl and C₁₋₄-hydroxyalkyl; each R₅ is independently selected from hydrogen, C₁₋₄-alkyl and C₁₋₄-hydroxyalkyl; m is 2, 3, 4 or 5; n is 2, 3, 4 or 5; and o is an integer from 1 to 10;
for removing carbon dioxide and/or hydrogen sulfide from fluid streams.

2. Absorbent for removing carbon dioxide and/or hydrogen sulfide from fluid streams, comprising an aqueous solution of a compound of the general formula I 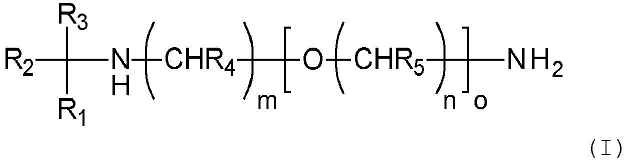 in which R₁, R₂ and R₃ are each independently selected from C₁₋₄-alkyl and C₁₋₄-hydroxyalkyl; each R₄ is independently selected from hydrogen, C₁₋₄-alkyl and C₁₋₄-hydroxyalkyl; each R₅ is independently selected from hydrogen, C₁₋₄-alkyl and C₁₋₄-hydroxyalkyl; m is 2, 3, 4 or 5; n is 2, 3, 4 or 5; and o is an integer from 1 to 10.

3. Compound according to Claim 2, wherein the R₅ radical on the carbon atom bonded directly to the primary amino group is hydrogen.

4. Absorbent according to either of Claims 2 and 3, wherein the absorbent comprises at least one organic solvent.

5. Absorbent according to any of Claims 2 to 4, wherein the absorbent comprises at least one acid.

6. Absorbent according to any of Claims 2 to 5, wherein the concentration of the compound of the formula (I) in the absorbent is 10% to 60% by weight.

7. Absorbent according to any of Claims 2 to 5, also comprising at least one tertiary amine and/or a sterically hindered primary or secondary amine.

8. Absorbent according to Claim 7, wherein the tertiary amine is methyldiethanolamine.

9. Process for removing carbon dioxide and/or hydrogen sulfide from fluid streams, wherein an absorbent of Claims 2 to 8 is contacted with a fluid stream.

10. Process according to Claim 9, wherein the fluid stream comprises hydrocarbons.

11. Process according to either of Claims 9 and 10, wherein the fluid stream has a total pressure of at least 3.0 bar.

12. Process according to any of Claims 9 to 11, comprising a regeneration step, wherein the regeneration step comprises at least one of the measures of heating, decompressing and stripping with an inert fluid.

13. Compound of the general formula (Ia) 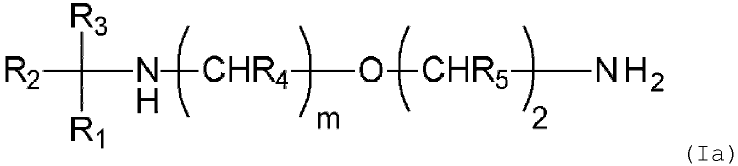 in which R₁, R₂ and R₃ are each independently selected from C₁₋₄-alkyl and C₁₋₄-hydroxyalkyl; each R₄ is independently selected from hydrogen, C₁₋₄-alkyl and C₁₋₄-hydroxyalkyl; each R₅ is independently selected from hydrogen, C₁₋₄-alkyl and C₁₋₄-hydroxyalkyl; and m is 2, 3, 4 or 5.

14. Compound according to Claim 13, with the proviso that the R₅ radical on the carbon atom bonded directly to the primary amino group is hydrogen.

15. Compound according to either of Claims 13 and 14, namely 2-(2-tert-butylaminoethoxy)ethylamine.

## Revendications

1. Utilisation d'un composé de formule générale (I) 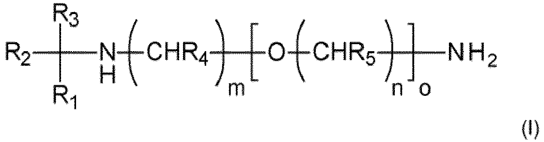
dans laquelle R₁, R₂ et R₃ sont choisis indépendamment les uns des autres parmi alkyle en C₁₋₄ et hydroxyalkyle en C₁₋₄ ; R₄ est choisi indépendamment à chaque fois parmi hydrogène, alkyle en C₁₋₄ et hydroxyalkyle en C₁₋₄ ; R₅ est choisi indépendamment à chaque fois parmi hydrogène, alkyle en C₁₋₄ et hydroxyalkyle en C₁₋₄ ; m représente 2, 3, 4 ou 5 ; n représente 2, 3, 4 ou 5 ; et o représente un nombre entier de 1 à 10 ;
pour l'élimination de dioxyde de carbone et/ou de sulfure d'hydrogène de courants fluides.

2. Agent d'absorption pour l'élimination de dioxyde de carbone et/ou de sulfure d'hydrogène de courants fluides, contenant une solution aqueuse d'un composé de formule générale (I) 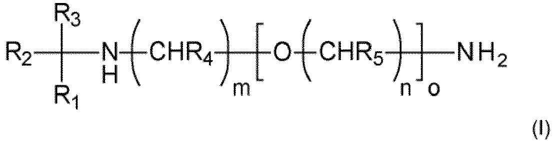 dans laquelle R₁, R₂ et R₃ sont choisis indépendamment les uns des autres parmi alkyle en C₁₋₄ et hydroxyalkyle en C₁₋₄ ; R₄ est choisi indépendamment à chaque fois parmi hydrogène, alkyle en C₁₋₄ et hydroxyalkyle en C₁₋₄ ; R₅ est choisi indépendamment à chaque fois parmi hydrogène, alkyle en C₁₋₄ et hydroxyalkyle en C₁₋₄ ; m représente 2, 3, 4 ou 5 ; n représente 2, 3, 4 ou 5 ; et o représente un nombre entier de 1 à 10.

3. Agent d'absorption selon la revendication 2, dans lequel le radical R₅ sur l'atome de carbone directement relié au groupe amino primaire représente hydrogène.

4. Agent d'absorption selon l'une quelconque des revendications 2 ou 3, dans lequel l'agent d'absorption comprend au moins un solvant organique.

5. Agent d'absorption selon l'une quelconque des revendications 2 à 4, dans lequel l'agent d'absorption comprend au moins un acide.

6. Agent d'absorption selon l'une quelconque des revendications 2 à 5, dans lequel la concentration du composé de formule (I) dans l'agent d'absorption est de 10 à 60 % en poids.

7. Agent d'absorption selon l'une quelconque des revendications 2 à 6, contenant en outre au moins une amine tertiaire et/ou une amine primaire ou secondaire à encombrement stérique.

8. Agent d'absorption selon la revendication 7, dans lequel l'amine tertiaire est la méthyldiéthanolamine.

9. Procédé d'élimination de dioxyde de carbone et/ou de sulfure d'hydrogène de courants fluides, dans lequel un agent d'absorption selon les revendications 2 à 8 est mis en contact avec un courant fluide.

10. Procédé selon la revendication 9, dans lequel le courant fluide contient des hydrocarbures.

11. Procédé selon l'une quelconque des revendications 9 ou 10, dans lequel le courant fluide présente une pression totale d'au moins 3,0 bar.

12. Procédé selon l'une quelconque des revendications 9 à 11, comprenant une étape de régénération, l'étape de régénération comprenant au moins une des mesures de chauffage, détente et extraction avec un fluide inerte.

13. Composé de formule générale (la) 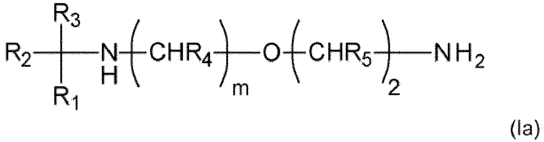 dans laquelle R₁, R₂ et R₃ sont choisis indépendamment les uns des autres parmi alkyle en C₁₋₄ et hydroxyalkyle en C₁₋₄ ; R₄ est choisi indépendamment à chaque fois parmi hydrogène, alkyle en C₁₋₄ et hydroxyalkyle en C₁₋₄ ; R₅ est choisi indépendamment à chaque fois parmi hydrogène, alkyle en C₁₋₄ et hydroxyalkyle en C₁₋₄ ; et m représente 2, 3, 4 ou 5.

14. Composé selon la revendication 13, à condition que le radical R₅ sur l'atome de carbone directement relié au groupe amino primaire représente hydrogène.

15. Composé selon l'une quelconque des revendications 13 ou 14, à savoir 2-(2-tert-butylaminoéthoxy)éthylamine.
